Europäisches Patentamt

(19) European Patent Office

Office européen des brevets

(11) Numéro de publication: **0 227 526**
**B1**

(12) FASCICULE DE BREVET EUROPEEN

(45) Date de publication du fascicule du brevet:
12.09.90

(51) Int. Cl.⁵: **A61L 15/00, A61L 15/16**

(21) Numéro de dépôt: 86402603.4

(22) Date de dépôt: 21.11.86

(54) Pansement pour le traitement des plaies et procédé de préparation.

(30) Priorité: 22.11.85 FR 8517361

(43) Date de publication de la demande:
01.07.87 Bulletin 87/27

(45) Mention de la délivrance du brevet:
12.09.90 Bulletin 90/37

(84) Etats contractants désignés:
AT BE CH DE ES FR GB GR IT LI LU NL SE

(56) Documents cités:
EP-A- 0 041 934
EP-A- 0 049 944
EP-A- 0 071 063
EP-A- 0 157 960
US-A- 3 767 784

CHEMICAL ABSTRACTS,
vol. 102, no. 16, 22 avril 1985, page 379, résumé
no. 137855b, Columbus, Ohio, US

Le dossier contient des informations techniques
présentées postérieurement au dépôt de la demande et
ne figurant pas dans le présent fascicule.

(73) Titulaire: BEGHIN-SAY SOCIETE ANONYME,
F-59239 Thumeries(FR)

(72) Inventeur: Lerailler, Eric, 8, Allée le Notre,
F-77200 Lognes(FR)
Inventeur: Pierre, Michel, 2, passage de l'Hôtel de Ville,
F-68100 Mulhouse(FR)
Inventeur: Thiriet, Bernard, 42, av. de la Terrasse,
F-91260 Juvisy/Orge(FR)
Inventeur: Wacquez, Jean, 4, av. du 11 Novembre,
F-92400 Courbevoie(FR)

(74) Mandataire: Quéré, Jean Pierre, BEGHIN-SAY Service
Propriété Industrielle 54, Avenue Hoche,
F-75008 Paris(FR)

ACTORUM AG

EP 0 227 526 B1

## Description

L'invention se rapporte à un procédé pour préparer une composition absorbant les liquides physiologiques: sécrétions de plaies, sang, etc. et à son application comme pansement dans le traitement des plaies.

Les produits absorbants auxquels il est fait référence par la suite sont des polymères, sous forme de macromolécules hydrophiles et pratiquement insolubles dans l'eau, qui gonflent en présence du liquide jusqu'à prendre la consistance d'un gel. Ces superabsorbants nommés également hydrogels, hydrorétenteurs ou additifs à rétention améliorée, en abrégé ARA, sont des solides capables ainsi de gélifier 10 à 1000 fois leur poids de liquide. Les gels obtenus ont la propriété de ne pas désorber les fluides facilement quand ils subissent une pression et que la masse est déformée. Pour cette raison, les superabsorbants trouvent de nombreuses applications en agriculture pour la rétention d'eau, et dans le domaine de l'hygiène pour l'absorption de liquides physiologiques tels que l'urine et le sang où ils sont employés comme additif pour améliorer la capacité d'absorption des masses absorbantes, masses fibreuses, généralement de la mousse de cellulose, constituant les couches, changes, serviettes périodiques et tampons. On les trouve aussi dans le domaine médical pour le traitement des plaies. Les produits les plus connus sont les alginates, les carboxyméthyl cellulose réticulés, les amidons greffés, les dérivés de synthèse du type acrylamide ou du type acrylate.

Ces produits présentent chacun des caractéristiques physicochimiques, telles que la vitesse de prise en gel et la capacité d'absorption qui leur sont propres. On constate cependant que ces caractéristiques, pour un même produit, varient considérablement suivant le liquide absorbé: eau pure, urine, sérum, sang.

Ainsi, la capacité d'absorption peut être divisée par 10 quand on passe de l'eau pure à l'eau salée; il en va de même pour la vitesse de prise en gel qui est fortement diminuée.

Par ailleurs, ces produits sont onéreux. Pour les articles d'hygiène à usage unique fabriqués en grandes séries, où les coûts sont calculés très serrés, il existe un besoin continu de recherche de produits plus économiques.

Dans le but d'améliorer les vitesses d'absorption du superabsorbant on a déjà cherché à les disposer à sec ou les disperser, dans des produits minéraux ou organiques en poudre, tels que la cellulose comme cela est décrit dans le brevet US 4 055 184, dans des matériaux inertes, tels que cellulose, argiles, brevets DE 2 264 027, JP 13 543 575, silice ou alumine, brevet us 3 932 322, ou la carboxyméthylcellulose, brevets us 4 043 921 et FR 2 488 901, dans des matériaux pulvérulents et solubles dans l'eau, EP 71 063, qui peuvent être des mono et/ou disaccharides JP 59 89 169.

L'invention a pour objet la préparation d'une composition absorbante à partir d'un superabsorbant quelconque, présentant des qualités améliorées par rapport au superabsorbant correspondant ou bien, présentant des qualités au moins aussi bonnes mais dont le coût est toujours inférieur ou dont le rapport efficacité/prix est amélioré.

L'invention a également pour objet l'application comme pansement dans le traitement des plaies, d'une composition absorbante préparée à partir d'un superabsorbant et d'au moins un oligosaccharide choisi parmi les disaccharides tels que saccharose, lactose, maltose, cellobiose, les monosaccharides tels que le glucose ou le fructose, les sucres supérieurs, les sirops de glucose.

L'invention s'applique à tous les superabsorbants connus mais en particulier elle s'est révélée donner des résultats significatifs avec des superabsorbants du type acrylate.

Le superabsorbant peut être choisi parmi une classe importante de produits, il s'agit de composés inorganiques (silicates...) ou organiques capables d'absorber puis de retenir un liquide même sous pression modérée. Parmi les composés organiques citons les alginates, les guars, agaragar, carraghénates et autres composés extraits de produits naturels ; les dérivés de cellulose dont les carboxyméthyl cellulose réticulés (exemple : AKUCELL de ENKA - AKZO/HOLLANDE), les hydroxyéthylcelluloses, produits non-ioniques insensibles aux solutions salines ; les dérivés d'amidon tels que les amidons polyacrylates (exemple : SANWET de SANYO CHEMICAL/JAPON) ; les polyacrylates de synthèse (exemples : AQUAKEEP de SEITETSU/JAPON ou LUQUASORB de BASF/ALLEMAGNE, obtenus par un procédé en suspension inverse, AQUALIC de NIPPON SHOKOBAI/JAPON et FAVOR de STOCKHAUSEN/ALLEMAGNE obtenus par un procédé en solution aqueuse), les copolymères polyacrylamides-polyacrylates ou encore les polyéthylenoxides, les polyvinylalcools, les polyvinyléthers, les copolymères d'anhydrides d'acides éthylenemaleiques, les polyvinylpyridines, polyvinylmorpholénones, les dérivés d'acides vinylsulfonique, acrylique, métacrylique etc...

Généralement ces macromolécules sont légèrement réticulées pour les rendre gonflables mais insolubles dans les solutions aqueuses. Elles sont réticulées par la chaleur (autoréticulation), par liaison covalente, ionique (sels di- ou trivalents), de Van der Waals ou liaison hydrogène.

Le sucre utilisé sera préférentiellement du saccharose pour sa facilité de mise en oeuvre et sa meilleure stabilité bactériologique par rapport à un sucre simple, mais d'autres sucres conviennent également.

Conformément à l'invention la composition est obtenue par mélange par voie humide. Il s'agit là d'un résultat surprenant de la synergie sirop de sucre-superabsorbant. En effet, compte tenu des interactions

2

évidentes eau-superabsorbant, les méthodes de mise en oeuvre de ces derniers évitent les phases liquides et plus particulièrement aqueuses. Or on a constaté que la composition pouvait être facilement obtenue par incorporation du superabsorbant dans une solution concentrée de sucre, saccharose notamment. De tels sirops de 60 à 80 Brix, voire des fondants patissiers, représentent des milieux solvants liquides, dans lesquels l'eau très fortement liée aux molécules de sucre, voit son activité thermodynamique diminuée. Dans ces conditions les interactions eau-superabsorbant sont complètement modifiées et il est possible de réaliser des pseudo-solutions homogènes de superabsorbant et de sucre ne gélifiant qu'après un certain temps de contact.

L'invention est caractérisée en ce que le procédé comprend les étapes suivantes :
- préparation d'une solution concentrée du mono- et/ou disaccharide dans l'eau
- incorporation du superabsorbant en quantité déterminée correspondant à la proportion de superabsorbant désirée dans la composition finale
- malaxage du mélange jusqu'à obtention d'une pâte homogène
- éventuellement séchage de la pâte jusqu'à une siccité de valeur déterminée

Tout le mono- et/ou disaccharide peut ne pas être apporté par la solution concentrée. Selon une forme préférée de l'invention une partie est apportée sous forme de poudre et est incorporée au mélange soit en même temps que le superabsorbant soit au cours du malaxage. Cette variante permet de mieux maîtriser la viscosité.

Selon les applications souhaitées, des additifs peuvent être ajoutés au mélange avant ou pendant la phase de malaxage en vue de lui conférer des activités spécifiques : oxydes de zinc, enzymes, acides aminés, acides ou sels organiques ou minéraux, vitamines, antibiotiques, antimicrobiens, tensio-actifs. On peut également ajouter des fibres textiles naturelles ou synthétiques qui augmenteront la vitesse d'absorption du liquide dans le mélange, par capillarité.

Le mélange sans addition de plastifiant, obtenu après séchage, est très dur et abrasif. Il peut être concassé, broyé, tamisé à la granulométrie adéquate.

L'addition de plastifiants ou de solvants à teneur contrôlée pour faciliter la mise en oeuvre technologique ou l'utilisation tels que la glycérine, le polyéthtylène-glycol ou l'alcool isopropylique par exemple fournissent des masses plastiques qui à 40 ou 50°C peuvent être laminées, extrudées, contre-collées sur un film ou une toile textile ou plastique, broyées à froid... Suivant la teneur en plastifiant, on obtient des produits qui à froid sont caractérisés par une plasticité rémanente, particulièrement souhaitable pour le traitement des plaies.

La composition est appliquée comme pansement absorbant pour le traitement des plaies par le sucre qu'elle contient.

En effet l'action cicatrisante du sucre est connue depuis longtemps qu'il s'agisse du simple sucre en poudre de cuisine ou du miel. Ses effets bénéfiques sont à attribuer essentiellement à son activité bactéricide et/ou bactériostatique, car en absorbant les sécrétions de la blessure, le sucre exerce une activité hydrique (Aw) trop basse pour permettre la croissance des germes présents dans la plaie. L'activité hydrique est définie comme le rapport de la pression de vapeur saturante de la solution à celle de l'eau pure. Pour une solution saturés de saccharose, par exemple, à température ambiante la valeur de Aw est de 0,86. Elle est inférieure à la valeur à laquelle les germes pathogènes généralement existant au niveau des plaies sont déjà inhibés. Ainsi les microorganismes responsables des infections ne peuvent se développer et la cicatrisation des tissus est plus rapide. La façon connue d'appliquer le sucre est très simple: après avoir nettoyé et séché la plaie avec une compresse, on verse le sucre dans la cavité que l'on a pris soin de bien ouvrir pour permettre sa bonne répartition. Un pansement sec adhésif est alors posé pour maintenir en place le sirop qui se forme.

La composition absorbante permet une mise en œuvre plus simple du traitement par l'action du superabsorbant qui se gélifie et fixe les exsudats avec une plus grande efficacité. Elle conduit à la formation d'une masse compacte. Cette masse, qu'il est possible de retirer facilement, rend la détersion des plaies et le changement des pansements plus aisés, elle améliore par conséquent le confort du malade et la rapidité des soins. Pour une application de ce type, le mélange comprendra entre 1% et 30% de superabsorbant pour obtenir une efficacité thérapeutique maximale.

D'autres détails sur le mode de préparation et des essais comparatifs sont exposés ci-après:

Ce mode de préparation peut être illustré par l'exemple suivant. On prélève une certaine quantité d'un sirop de saccharose pur à 80 Brix et suffisamment chaud pour ne pas recristalliser, soit pour 500 g de sirop, 400 g de sucre et 100 g d'eau. On verse le sirop chaud dans un malaxeur (par exemple du type Guedu) à double enveloppe chauffée, comportant une hélice en forme de Z. On peut ajouter un plastifiant, (dans notre exemple 37,5 g de glycérol) qui contribue à diminuer la viscosité du milieu ainsi que l'activité de l'eau. Toujours sous agitation, on ajoute en une fois le superabsorbant, soit dans notre exemple 50 g de produit vendu sous la marque SANWET, référencé IM1000, par SANYO CHEMICAL/JAPON. En quelques secondes on obtient une masse épaisse, translucide légèrement collante. Pour terminer la mise en forme du composé, et obtenir les proportions sucre-superabsorbant désirées, compte tenu des conditions imposées par le type de séchage, il peut être nécessaire de rajouter du sucre, dans notre exemple on verse 100 g de sucre glace non amylacé. On laisse tourner l'hélice du malaxeur encore pendant quelques secondes jusqu'à obtention d'un bloc homogène plastique et non collant. Le bloc peut être extrudé à travers une filière ou bien laminé en forme de plaque sur un marbre, ou encore pressé entre les mâ-

EP 0 227 526 B1

choires d'une presse hydraulique. On sèche ensuite le produit, par exemple en étuve jusqu'à obtenir un taux de matière sèche compris entre 85% et 98% et de préférence de 92% à 96%.

Dans le cas où l'on travaille avec une quantité suffisamment importante de plastifiant, on obtient après séchage modéré une masse suffisamment plastique pour être travaillée ou granulée par exemple dans un granulateur de type FREWITT.

Une forme préférée de l'invention consiste à réaliser un mélange à base de sucre et de superabsorbant et à l'enduire sur un support textile. Après séchage à l'étuve on obtient un composite souple pouvant être découpé mécaniquement et utilisé comme pansement de plaies.

Pour cette application, la méthode permet l'incorporation homogène de produits chimiques ou pharmaceutiques utiles au traitement des plaies :
. oxyde de zinc, jusqu'à environ 10 % de la masse totale
. bioxyde de zinc, jusqu'à environ 10 % de la masse totale
. des produits surfactants, jusqu'à environ 0,2 % de la masse totale
. des enzymes en particulier des protéases, des acides aminés, acides organiques ou minéraux ou leurs sels, des vitamines, antibiotiques, antimicrobiens, bactériostatiques, colorants, parfums, etc...

Si l'on incorpore un plastifiant, il peut s'agir, en proportion de la masse totale, de :
. glycérol, déjà mentionné, jusqu'à environ 15 %
. polyvinyl alcool, jusqu'à environ 10 %
. polyvinyl-pyrrolidone, jusqu'à environ 15 %
. polyéthylène glycol, jusqu'à environ 10 %
. et plus généralement, polyols liquides, jusqu'à environ 10 %

Durant la préparation on peut également ajouter un diluant volatil, isopropanol par exemple qui abaisse la viscosité et facilite la dispersion et la mise en oeuvre de la masse plastique. Le diluant sera éliminé en grande partie lors du séchage.

Le sucre utilisé est de préférence le saccharose mais il peut lui-même comporter d'autres types de sucre par exemple un sirop de glucose entre 0 et 15 %, du sorbitol, entre 0 et 10 %, du lactose,...

Le procédé de préparation conforme à l'invention peut également être mis en oeuvre de façon à permettre une production en continu de la composition absorbante. On utilise à cette fin une extrudeuse qui peut être de type bi-vis. On introduit à débit régulier, entre les vis de l'extrudeuse, le sirop de sucre saturé à 60-80 Brix, additionné d'une certaine quantité de plastifiant, glycérol par exemple. On introduit simultanément dans la trémie d'alimentation, le superabsorbant en poudre, mélangé à sec avec du sucre glace.

Par ses conditions de fonctionnement très souples, l'extrudeuse ne soumet la matière en mouvement qu'à de faibles contraintes de cisaillement n'entraînant pas de détérioration des qualités du superabsorbant. Le temps de séjour du mélange à l'intérieur de l'extrudeuse est compris entre 15 et 60 secondes. Des éléments chauffants peuvent être prévus afin de faciliter la plastification et contrôler la viscosité, toutefois cette dernière ne doit pas être réduite à un point où le produit deviendrait trop fluide.

En sortie d'extrudeuse, on obtient un mélange homogène qui peut être laminé par passage entre des cylindres, et ensuite associé à un support quelconque. Eventuellement une filière mono- ou multitrous peut être adaptée à l'extrémité aval de l'extrudeuse pour obtenir le produit sous forme de filaments. En aval du moyen de mise en forme du mélange un dispositif de chauffage approprié par rayonnement infrarouge, ou micro-ondes peut être prévu pour l'amener au taux de siccité souhaité.

On a réalisé de la sorte un mélange homogène à partir de 20 kg de produits de départ dont les proportions étaient les suivantes :

| mélange à sec: | – sucre glace | 9,3 kg |
| | – superabsorbant | 1,11 kg |
| sirop: | – saccharose | 5,5 kg |
| | – eau | 2,97 kg |
| | – glycérol | 1,11 kg |

Pour préciser et quantifier les propriétés des produits obtenus par le procédé de l'invention, des essais comparatifs ont été réalisés pour évaluer leur propriété d'absorption d'eau salée (Nacl 10 g/l + surfactif à 150 PPM), d'un liquide comparable au sérum et à la lymphe (par exemple un soluté injectable pour perfusion vendu sous la marque PLASMION, LABORATOIRE R. BELLON) et d'une formule sanguine (de composition, sang de bovin 85 %, eau salée 15 % à 9 g/l de Nacl, avec addition d'un anticoagulant, héparinate de sodium 0,025 g/l). En fonction de l'origine du sang de bovin, la composition physiologique varie et les résultats des essais menés par série comparative en tiennent compte sous le nom de Formule A, Formule B, Formule C.

Les tests d'évaluation d'absorption sont les suivants :

4

I) Rétention R30

Cette méthode permet d'évaluer la capacité de rétention d'un liquide par un superabsorbant. Pendant 30 minutes, on immerge dans le liquide une serviette périodique contenant 0,5 gramme de superabsorbant pur dispersé dans des quantités variables de sucre et éventuellement d'additifs. Après centrifugation à 1100 gravités pendant 60 secondes, la quantité de liquide absorbé par l'échantillon est calculée par différence entre l'absorption d'une serviette témoin sans échantillon et celle de la serviette contenant l'échantillon. La rétention R30 est exprimée en grammes de liquide absorbé par gramme de superabsorbant.

Pour tous ces calculs, on a considéré que le sucre ne se dissolvait pas dans la phase liquide (saline ou sanguine), il en résulte que les valeurs R30 données sont des valeurs minimales théoriques.

II) Test de prise en gel, (test du vortex)

La méthode de prise en gel est une mesure de la vitesse et de la capacité maximale d'absorption d'un gel de superabsorbant. Dans un bécher de 150 ml (diamètre 55 mm) contenant 100 ml de liquide de test à une température donnée, agité par un barreau aimanté (longueur 45 mm, Ø 8 mm) en rotation à 600 tour/mn, on verse instantanément une masse déterminée d'échantillon et on mesure le temps au bout duquel le vortex disparaît. On réalise successivement des essais avec des masses d'échantillon variables. L'absorption par prise en gel est fonction du temps selon une loi expérimentale du type :

$$\frac{1}{a} = \frac{1}{G} + \frac{1}{V_o(t - t_o)}$$

Les lettres ont les significations suivantes :
a = absorption en g/g
G = capacité maximale d'absorption en g/g
$V_o$ = vitesse initiale de prise en gel en g/g/s
$t_o$ = temps de retard de la prise en gel ou temps de mouillage en seconde

Toutes les valeurs sont rapportées à 1 g de superabsorbant contenu dans les échantillons.

Un programme de calcul par régression sélectionne le meilleur élément de corrélation en fonction d'hypothèses sur la valeur de G. Il permet de sélectionner les valeurs optimales des coefficients G, $V_o$ et $t_o$ qui caractérisent la prise en gel.

Pour illustrer la présente invention on a réalisé les produits suivants et leurs propriétés ont été testées par les méthodes d'évaluation d'absorption décrites ci-dessus.

EXEMPLE I

On a réalisé des mélanges, de saccharose et de superabsorbant AQUAKEEP.

Les mélanges sont séchés à l'étuve sous vide, ensuite broyés et tamisés. La fraction 100 - 500 microns est soumise aux tests.

Test de rétention R30

## TABLEAU 1a

| SACCHAROSE % | | AQUAKEEP % | | EAU SALEE 35°C g/g | | FORMULES SANGUINES 35°C | |
|---|---|---|---|---|---|---|---|
| | | | | | | A g/g | B g/g |
| 100 | ! | 0 | ! | 0 | ! | 0 | 0 |
| 93,5 | ! | 6,5 | ! | - | ! | 18 | 13,5 |
| 90,9 | ! | 9,1 | ! | 47 | ! | 16,5 | 11 |
| 85 | ! | 15 | ! | - | ! | - | 12 |
| 80 | ! | 20 | ! | 47 | ! | - | 11 |
| 70 | ! | 30 | ! | 41 | ! | - | 12 |
| 0 | ! | 100 | ! | 49 | ! | 12 | 7 |

Le mélange par voie humide montre une synergie de plus de 50 % pour l'absorption du sang.

Pour l'absorption de l'eau salée, les performances du superabsorbant pur sont maintenues. L'absence de synergie avec l'eau salée montre que la synergie avec le sang n'est pas liée à un phénomène d'accessibilité physique de liquide sur l'échantillon. Par ailleurs les mesures de rétention R30 dans l'eau salée sont caractéristiques du superabsorbant et de sa teneur dans l'échantillon.

Test du vortex

## TABLEAU 1b

| SACCHAROSE % | | AQUAKEEP % | | EAU SALEE 35°C | | | PLASMION 35°C | | | FORMULES SANGUINES 35°C B | | | C | | |
|---|---|---|---|---|---|---|---|---|---|---|---|---|---|---|---|
| | ! | | ! | G | Vo | To | G | Vo | To | G | Vo | To | G | Vo | To |
| 93,5 | ! | 6,5 | ! | 300 | 5,8 | 8 | 145 | 3,7 | 6 | 183 | 2,9 | 0 | 243 | 4,2 | 0 |
| 90,9 | ! | 9,1 | ! | 220 | 5,9 | 0 | 115 | 6,6 | 7 | 354 | 4 | 0 | 323 | 4,3 | 0 |
| 85 | ! | 15 | ! | 192 | 14 | 12 | 130 | 8,1 | 9,5 | 138 | 6 | 13 | 254 | 2,5 | 0 |
| 80 | ! | 20 | ! | - | - | - | - | - | - | 149 | 2,9 | 0 | 315 | 2,7 | 0 |
| 70 | ! | 30 | ! | 164 | 24 | 4 | 148 | 7,5 | 3 | - | - | - | - | - | - |
| 0 | ! | 100 | ! | 124 | 16 | 0,5 | 126 | 9,6 | 1 | 120 | 2,5 | 0 | 128 | 2,8 | 14 |

Le tableau 1b rassemble les valeurs de G, Vo, To pour différents mélanges respectivement pour l'eau salée, le PLASMION et la formule sanguine C, relevées lors du test de prise en gel effectué à 35°C.

Si on compare les synergies sucre-superabsorbant pour l'absorption des formules sanguines, on observe que le mélange par voie humide a tendance à augmenter la vitesse Vo. On constate une synergie pour le sang et dans une moindre mesure pour l'eau salée, quand on considère la capacité maximale d'absorption, G.

Le PLASMION donne des valeurs intermédiaires entre eau salée et sang.

Exemple II

Afin de vérifier les propriétés de la composition pour différents types de superabsorbant on a préparé des mélanges de saccharose et d'un superabsorbant, respectivement un amidon polyacrylate vendu sous la marque SANWET, un polyacrylate de synthèse obtenu par un procédé en solution aqueuse vendu sous la marque AQUALIC, un carboxyméthyl cellulose réticulé vendu sous la marque AKUCELL, un polyacrylate de synthèse obtenu par un procédé en suspension inverse vendu sous la marque LUQUASORB, et un deuxième acrylate de synthèse obtenu par un procédé en solution aqueuse vendu sous la marque FAVOR.

Les mélanges sont séchés, broyés et tamisés comme pour l'exemple I.

Test de rétention R30 en formules sanguines C et B

## TABLEAU 2a

| SACCHAROSE % | ARA % | SANWET C | SANWET B | AQUALIC C | AKUCELL C | LUQUASORB C |
|---|---|---|---|---|---|---|
| 93,5 | 6,5 | - | 13,5 | - | - | - |
| 90,9 | 9,1 | - | 15 | - | - | - |
| 80 | 20 | 12 | 9 | 11 | 11,5 | 16,5 |
| 70 | 30 | 11 | 11 | 8 | 11,5 | 13,5 |
| 0 | 100 | 6,5 | 6,5 | 4,5 | 13,5 | 8,5 |

## Test de Vortex

## TABLEAU 2b

| SACCHAROSE % | SANWET % | EAU SALEE 35°C G | Vo | To | PLASMION 35°C G | Vo | To | FORMULES SANGUINES 35°C B G | Vo | To | C G | Vo | To |
|---|---|---|---|---|---|---|---|---|---|---|---|---|---|
| 93,5 | 6,5 | 60 | 1,4 | 0 | 60 | 1,7 | 1 | 130 | 2,3 | 0 | - | - | - |
| 90,4 | 9,1 | 94 | 3,6 | 0 | 82 | 6,2 | 9 | 164 | 5 | 8 | 180 | 4 | 0 |
| 85 | 15 | 132 | 2,9 | 0 | 115 | 3,4 | 2 | 126 | 3,9 | 6,5 | - | - | - |
| 80 | 20 | 108 | 3,5 | 0 | 76 | 3,8 | 4 | 123 | 2,7 | 0 | 150 | 2,2 | 0 |
| 70 | 30 | - | - | - | 94 | 3,4 | 8 | 100 | 3,0 | 11 | - | - | - |
| 0 | 100 | 114 | 3,5 | 0 | 86 | 2,1 | 0 | 72 | 0,5 | 0 | 80 | 0,4 | 0 |

De ces tableaux il ressort que la synergie pour le sang est encore démontrée, quel que soit le superabsorbant employé, tant au niveau des valeurs des R30 que des valeurs de G.

Exemple III

Dans cet exemple on vérifie l'effet que peut produire l'incorporation d'un additif sur les propriétés du mélange.

On peut avoir recours à l'adjonction d'additifs à certaines associations saccharose-superabsorbant en fonction de 2 objectifs distincts :

1) Faciliter la dispersion du superabsorbant au sein du sirop de sucre et améliorer les qualités rhéologiques de la composition au sortir du mélangeur, ses qualités plastiques après séchage.

2) Conférer au mélange des qualités spécifiques correspondant à l'usage auquel on destine le composé, en particulier dans le domaine du traitement des plaies.

L'incorporation du glycerol, isopropanol et polyéthylène-glycol par exemple correspond au 1er objectif.

On a réalisé 29 préparations par voie humide de saccharose et superabsorbant comprenant divers additifs. Les compositions sont reprises dans le tableau 3a. Les proportions d'additifs mentionnées correspondent aux valeurs réelles au début de la mise en oeuvre du mélange.

Ces 29 préparations ont été soumises au test de rétention R30 pour l'eau salée et les formules sanguines, et au test de prise en gel, vortex pour l'eau salée, le plasmion et les formules sanguines. Les résultats sont repris dans les tableaux 3b et 3c.

Le tableau 3b montre qu'on peut incorporer jusqu'à 6,5 % de glycerol aux associations saccharose-AQUAKEEP (voir les produits 4 et 5 par rapport au produit 3) et saccharose-SANWET (voir les produits 11 et 12 par rapport au produit 10 et le produit 13 par rapport au produit 14) en maintenant les niveaux de rétention sanguine du composé sans additif.

Le tableau 3c montre une augmentation du pouvoir gélifiant théorique G, pour le sang et l'eau salée, comme dans les exemples précédents.

Le produit n° 5 en particulier, sous forme de granulé ou de feuilles répond bien aux spécifications de capacité d'absorption pour usage dans le traitement des plaies en milieu hospitalier ou domestique.

De même, l'isopropanol peut être incorporé jusqu'à 8 % (voir n°19 par rapport au n° 3 et n° 20 par rapport au n° 10), le mélange glycérol-isopropanol jusqu'à un total de 9 % (n° 9 par rapport au n° 3, n° 16 par rapport au n° 10) et le polyéthylène-glycol jusqu'à 8 % (n° 21-23 par rapport au n° 3, n° 22-24 par rapport au n° 10) sans affecter de manière sensible les performances des associations saccharose-superabsorbant correspondantes.

L'incorporation d'additifs ayant une action curative tels que : oxydes de zinc, acides à action détersive, antiseptique ou biochimique, (par exemple acide lactique, acide sorbique, borique...), cicatrisants vitaminiques et autres, extraits biologiques ou végétaux, aminoacides, antiseptiques du type povidone ou autres, sulfamides, antibiotiques, tensioactifs, etc... sont sans effet apparent sur les performances des associations simples saccharose-superabsorbant lorsque l'on reste dans les teneurs habituelles recommandées par la pharmacopée.

Une réalisation particulièrement avantageuse de l'invention consiste à adjoindre au mélange une protéase dont les effets sont spécifiquement à attendre au niveau du traitement des plaies et du sang. La composition n° 29 répond à cette formule et manifeste une activité renforcée par rapport au produit n° 10. On notera que pendant le processus de fabrication on a constaté expérimentalement le maintien à 70 % du pouvoir protéolytique de l'enzyme mise en oeuvre au départ.

## TABLEAU 3a

| N° | SACCHAROSE % | AQUAKEEP (AK) SANWET (SW) % | | ADDITIFS % | |
|---|---|---|---|---|---|
| 1 | 0 | AK | 100 | - | |
| 2 | 0 | SW | 100 | - | |
| 3 | 90,9 | AK | 9,1 | - | |
| 4 | 87 | AK | 8,7 | GLYCEROL | 4,3 |
| 5 | 85,1 | AK | 8,5 | " | 6,4 |
| 6 | 73,6 | AK | 20 | " | 6,4 |
| 7 | 80 | AK | 20 | - | |
| 8 | 87 | AK | 8,7 | ISOPROPANOL | 4,3 |
| 9 | 82,9 | AK | 8,3 | GLYCEROL | 4,4 |
| | | | | ISOPROPANOL | 4,4 |
| 10 | 90,9 | SW | 9,1 | - | |
| 11 | 87 | SW | 8,7 | GLYCEROL | 4,3 |
| 12 | 85,1 | SW | 8,5 | " | 6,4 |
| 12 | 73,6 | SW | 20 | " | 6,4 |
| 14 | 80 | SW | 20 | - | |
| 15 | 87 | SW | 8,7 | ISOPROPANOL | 4,3 |
| 16 | 82,9 | SW | 8,3 | GLYCEROL | 4,4 |
| | | | | ISOPROPANOL | 4,4 |
| 17 | 88,9 | AK | 8,9 | ISOPROPANOL | 2,2 |
| 18 | 88,9 | SW | 8,9 | " | 2,2 |
| 19 | 83,3 | AK | 8,3 | " | 8,3 |
| 20 | 83,3 | SW | 8,3 | " | 8,3 |
| 21 | 88,9 | AK | 8,9 | PEG 300 | 2,2 |
| 22 | 88,9 | SW | 8,9 | " | 2,2 |
| 23 | 83,3 | AK | 8,3 | " | 8,3 |
| 24 | 83,3 | SW | 8,3 | " | 8,3 |
| 25 | 93,5 | AK | 6,5 | - | |
| 26 | 93,5 | SW | 6,5 | - | |
| 27 | 82,3 | AK | 5,4 | PEG 300 | 4,6 |
| | | | | ZnO | 7,7 |
| 28 | 82,3 | SW | 5,4 | PEG 300 | 4,6 |
| | | | | ZnO | 7,7 |
| 29 | 90,7 | SW | 9,1 | PROTEASE | 0,18 |

## TABLEAU 3b

### Test de rétention R30

| N° | EAU SALEE 35°C | RETENTIONS FORMULES SANGUINES 35°C | | |
|---|---|---|---|---|
| | | A | B | C |
| 1 | 49 | 12 | 7 | 6 |
| 2 | 44 | - | 6,5 | - |
| 3 | 47 | 16,5 | 11 | - |
| 4 | - | - | - | - |
| 5 | - | - | - | - |
| 6 | - | - | 11 | - |
| 7 | 47 | - | 11 | - |
| 8 | - | - | - | - |
| 9 | - | 17,2 | - | - |
| 10 | - | - | 15 | - |
| 11 | - | 14 | - | - |
| 12 | - | 11 | - | - |
| 13 | - | - | 11 | - |
| 14 | 41,5 | - | 9 | 12 |
| 15 | - | 22,7 | - | - |
| 16 | - | 16 | - | - |
| 17 | - | 18,5 | - | - |
| 18 | - | 19 | 17,7 | - |
| 19 | - | - | - | - |
| 20 | - | - | - | - |
| 21 | - | - | - | - |
| 22 | - | - | - | - |
| 23 | - | - | - | - |
| 24 | - | - | - | - |
| 25 | - | 18 | 13,5 | - |
| 26 | - | - | 13,5 | - |
| 27 | - | - | - | - |
| 28 | - | - | - | - |
| 29 | - | 19 | - | - |

## TABLEAU 3c

Test de prise en gel, vortex

| N° | EAU SALEE 35°C | | | PLASMION 35°C | | | VORTEX FORMULES SANGUINES 35°C A | | | B | | | C | | |
|---|---|---|---|---|---|---|---|---|---|---|---|---|---|---|---|
| | G | Vo | To | G | Vo | To | G | Vo | To | G | Vo | To | G | Vo | To |
| 1 | 124 | 16 | 0,5 | 126 | 9,6 | 1 | - | - | - | 120 | 2,5 | 0 | 128 | 2,8 | 14 |
| 2 | 114 | 3,5 | 0 | 86 | 2,1 | 0 | - | - | - | - | - | - | 76 | 0,4 | 0 |
| 3 | 220 | 5,9 | 0 | 115 | 6,6 | 7 | - | - | - | 354 | 4 | 0 | 323 | 4,3 | 0 |
| 4 | 216 | 7 | 8 | 119 | 9,6 | 1,8 | 247 | 4,8 | 0 | - | - | - | - | - | - |
| 5 | 188 | 13,4 | 1 | 109 | 9,1 | 0 | - | - | - | - | - | - | - | - | - |
| 6 | 186 | 8,2 | 0 | 128 | 6,7 | 7 | - | - | - | 237 | 2,4 | 0 | - | - | - |
| 7 | - | - | - | - | - | - | - | - | - | 149 | 2,9 | 0 | 315 | 2,7 | 0 |
| 8 | 206 | 10,9 | 8 | 250 | 2,7 | 0 | - | - | - | - | - | - | - | - | - |
| 9 | 147 | 24,1 | 14 | 212 | 2,8 | 0 | 423 | 2,2 | 0 | - | - | - | - | - | - |
| 10 | 94 | 3,6 | 0 | 82 | 6,2 | 9 | - | - | - | 164 | 5 | 8 | 180 | 4 | 0 |
| 11 | 132 | 4,3 | 0 | 104 | 9,4 | 13 | - | - | - | - | - | - | - | - | - |
| 12 | 154 | 6,2 | 0 | 121 | 5,2 | 2,1 | - | - | - | - | - | - | - | - | - |
| 13 | 136 | 3,4 | 0 | 91 | 3,3 | 2 | - | - | - | - | - | - | 69 | 122 | 50 |
| 14 | 108 | 3,5 | 0 | 76 | 3,8 | 4 | - | - | - | 123 | 2,7 | 0 | 150 | 2,2 | 0 |
| 15 | 121 | 2,9 | 0 | 70 | 5,4 | 10 | 226 | 3,6 | 0 | - | - | - | - | - | - |
| 16 | 165 | 1 | 0 | 88 | 1,9 | 5 | 99 | 1,2 | 0 | - | - | - | - | - | - |
| 17 | 235 | 4,4 | 0 | 130 | 3,6 | 2,5 | 184 | 5,9 | 7 | - | - | - | - | - | - |
| 18 | 60 | 1,6 | 0 | 75 | 1,5 | 12 | 196 | 2,8 | 1,7 | - | - | - | - | - | - |
| 19 | 242 | 7,8 | 0 | 134 | 6,1 | 0 | - | - | - | - | - | - | - | - | - |
| 20 | 94 | 5,3 | 0 | 73 | 4,3 | 0 | - | - | - | - | - | - | - | - | - |
| 21 | 347 | 7,3 | 0 | 190 | 3,1 | 0 | - | - | - | - | - | - | - | - | - |
| 22 | 75 | 1,3 | 0 | 132 | 2,1 | 3 | - | - | - | - | - | - | - | - | - |
| 23 | 354 | 4,3 | 0 | 179 | 3,8 | 0 | - | - | - | - | - | - | - | - | - |
| 24 | 58 | 1,3 | 0 | 83 | 0,6 | 0 | - | - | - | - | - | - | - | - | - |
| 25 | 300 | 5,8 | 8 | 145 | 3,7 | 6 | - | - | - | 183 | 2,9 | 0 | 243 | 4,2 | 0 |
| 26 | 60 | 1,4 | 0 | 60 | 1,7 | 1 | - | - | - | 130 | 2,3 | 0 | - | - | - |
| 27 | 146 | 6,8 | 0,8 | 115 | 8,6 | 1,2 | - | - | - | - | - | - | - | - | - |
| 28 | 62 | 4,2 | 0 | 58 | 3 | 4,1 | - | - | - | - | - | - | - | - | - |
| 29 | 163 | 7,5 | 0 | 116 | 9,5 | 5 | - | - | - | - | - | - | - | - | - |

## Revendications

1. Pansement pour le traitement des plaies caractérisé en ce qu'il est composé essentiellement d'au moins un superabsorbant et d'un mono- ou disaccharide, mélangés en phase humide, la quantité de superabsorbant dans le mélange étant comprise entre 1% et 30%.

2. Pansement selon la revendication 1 caractérisé en ce que la disaccharide est le saccharose.

3. Pansement selon l'une des revendications 1 et 2 caractérisé en ce qu'il comprend en outre jusqu'à 10% d'un plastifiant.

4. Pansement selon la revendication 3 caractérisé en ce que le plastifiant est un polyol tel que glycérol, PEG, PVA ou polyvinyl pyrrolidone.

5. Pansement selon l'une des revendications 1 à 4 caractérisé en ce qu'il se présente sous la forme d'une pâte extrudée sur un support textile.

6. Pansement selon l'une des revendications 1 à 4 caractérisé en ce qu'il se présente sous la forme de granulés ou de filaments.

7. Procédé de fabrication d'un pansement selon l'une des revendications précédentes caractérisé en ce qu'il comprend les étapes suivantes:
   – préparation d'une solution concentrée du mono- et/ou disaccharide dans l'eau
   – addition d'une quantité déterminée de superabsorbant correspondant à la quantité désirée de superabsorbant dans le produit final
   – malaxage des composants jusqu'à l'obtention d'une pâte homogène
   – éventuellement séchage de la pâte de manière à ramener la teneur en eau du produit à une valeur comprise entre 2 et 15%.

8. Procédé selon la revendication 7, caractérisé en ce qu'un plastifiant ou un diluant, dans une proportion inférieure à 10% de la masse totale, est ajouté au mélange avant l'opération de malaxage, ou lors de la préparation du sirop de sucre.

9. Procédé selon la revendication 8, caractérisé en ce que le plastifiant ou diluant est pris dans le groupe: glycérol, PEG, PVA, isopropanol, polyvinyl pyrrolidone, polyols.

10. Procédé selon l'une des revendications 7 à 9, caractérisé en ce qu'un additif pharmaceutique est incorporé au mélange.

11. Procédé selon l'une des revendications 7 à 10, caractérisé en ce que des fibres textiles naturelles ou synthétiques sont incorporées au mélange, au cours de la phase de malaxage notamment.

12. Procédé selon l'une des revendications 7 à 11, caractérisé en ce qu'une partie du sucre est incorporée à sec au mélange, sous forme de poudre.

13. Procédé selon la revendication 12, caractérisé en ce que ledit sucre à sec est prémélangé au superabsorbant avant le mélange de ce dernier avec la solution concentrée.

14. Procédé selon l'une des revendications 7 à 13, caractérisé en ce que le produit obtenu est appliqué sur un support de manière à former une structure composite.

## Patentansprüche

1. Verband zur Behandlung von Wunden, dadurch gekennzeichnet, daß er im wesentlichen aus mindestens einem Superabsorptionsmittel und einem Mono- oder Disaccharid besteht, die in feuchter Phase miteinander vermischt worden sind, und wobei der Anteil des Superabsorptionsmittels in dem Gemisch zwischen 1% und 30% beträgt.

2. Verband nach Anspruch 1, dadurch gekennzeichnet, daß das Disaccharid Saccharose ist.

3. Verband nach einem der Ansprüche 1 und 2, dadurch gekennzeichnet, daß er darüber hinaus bis zu 10% eines Weichmachers enthält.

4. Verband nach Anspruch 3, dadurch gekennzeichnet, daß es sich bei dem Weichmacher um ein Polyol wie Glyzerol, PEG, PVA oder Polyvinylpyrrolidon handelt.

5. Verband nach einem der Ansprüche 1 bis 4, dadurch gekennzeichnet, daß er sich in Form einer auf einem Textilträger extrudierten Masse darstellt.

6. Verband nach einem der Ansprüche 1 bis 4, dadurch gekennzeichnet, daß er in gekörnter oder fertiger Form vorliegt.

7. Verfahren zur Herstellung eines Verbandes nach einem der vorhergehenden Ansprüche, dadurch gekennzeichnet, daß es folgende Schritte aufweist:
   – Herstellung einer konzentrierten wässrigen Lösung eines Mono- und/oder Disaccharides
   – Zugabe einer bestimmten dem gewünschten Anteil des Superabsorptionsmittels in dem Endprodukt entsprechenden Menge an Superabsorptionsmittel
   – Vermischen der Bestandteile bis zum Erhalt einer homogenen Masse
   – ggf. Trockung der Masse derart, daß der Wassergehalt des Produktes auf einen Wert zwischen 2 und 15% erniedrigt wird.

8. Verfahren nach Anspruch 7, dadurch gekennzeichnet, daß ein Weichmacher oder ein Verdünnungsmittel dem Gemisch in einem Anteil geringer als 10% der Gesamtmasse vor der Mischapparation oder während der Herstellung des Zuckersirups zugefügt wird.

9. Verfahren nach Anspruch 8, dadurch gekennzeichnet, daß der Weichmacher oder das Verdünnungsmittel aus der Gruppe: Glyzerol, PEG, PVA, Isopropanol, Polyvinylpyrrolidon, Polyole, gewählt wird.

10. Verfahren nach einem der Ansprüche 7 bis 9, dadurch gekennzeichnet, daß ein pharmazeutisches Additiv dem Gemisch zugesetzt wird.

11. Verfahren nach einem der Ansprüche 7 bis 10, dadurch gekennzeichnet, daß natürliche oder synthetische Textilfasern dem Gemisch insbesondere während der Mischphase zugesetzt werden.

12. Verfahren nach einem der Ansprüche 7 bis 11, dadurch gekennzeichnet, daß ein Teil des Zuckers trocken in Form von Puder in das Gemisch eingearbeitet wird.

13. Verfahren nach Anspruch 12, dadurch gekennzeichnet, daß der erwähnte trockene Zucker mit dem Superabsorptionsmittel vorgemischt wird, bevor das letztere mit der konzentrierten Lösung gemischt wird.

14. Verfahren nach einem der Ansprüche 7 bis 13, dadurch gekennzeichnet, daß das erhaltene Produkt auf einem Träger aufgebracht wird derart, daß eine Verbundstruktur gebildet wird.

## Claims

1. A dressing for the treatment of wounds characterised in that it consists essentially of a superabsorbent and monosaccharide or disaccharide mixed in the wet phase, the quantity of superabsorbent in the mixture lying between 1% and 30%.

2. A dressing according to claim 1 characterised in that the disaccharide is saccharose.

3. A dressing according to one of claims 1 and 2 characterised in that it also contains up to 10% of a softener.

4. A dressing according to claim 3 characterised in that the softener is a polyol such as glycerol, PEG, PVA or polyvinyl pyrrolidone.

5. A dressing according to one of claims 1 to 4 characterised in that it takes the form of a paste extruded onto a textile backing.

6. A dressing according to one of claims 1 to 4 characterised in that it is in the form of granules or filaments.

7. A process for the manufacture of a dressing according to one of the foregoing claims characterised in that it comprises the following stages:
   – preparation of a concentrated solution of the monosaccharide and/or disaccharide in water
   – addition of a specific quantity of superabsorbent corresponding to the desired quantity of superabsorbent in the final product
   – mixing of the components until a homogeneous paste is obtained
   – drying of the paste if applicable in such a way as to reduce the water content of the product to a value between 2 and 15%.

8. A process according to claim 7, characterised in that a softener or a diluent is added to the mixture in a proportion less than 10% of the total mass before the mixing operation or during the preparation of the sugar syrup.

9. A process according to claim 8, characterised in that the softener or diluent is taken from the group: glycerol, PEG, PVA, isopropanol, polyvinyl pyrrolidone, polyols.

10. A process according to one of claims 7 to 9, characterised in that a pharmaceutical additive is incorporated into the mixture.

11. A process according to one of claims 7 to 10, characterised in that natural or synthetic textile fibres are incorporated into the mixture, in particular during the mixing stage.

12. A process according to one of claims 7 to 11, characterised in that part of the sugar is incorporated in the mixture dry, in the form of powder.

13. A process according to claim 12, characterised in that the said dry sugar is premixed with the superabsorbent before the latter is mixed with the concentrated solution.

14. A process according to one of claims 7 to 13, characterised in that the product obtained is applied to a backing in such a way as to form a composite structure.